# EUROPEAN PATENT APPLICATION

(11) **EP 4 279 091 A1**
(43) Date of publication of application: **22.11.2023**
(21) Application number: 22739828.6
(22) Date of filing: 18.01.2022
(51) Int. Cl.: A61K 48/00, A61K 31/7088, A61P 9/10

(54) **COMPOSITION COMPRISING MIR 145 INHIBITOR AS ACTIVE INGREDIENT FOR TREATMENT OF MYOCARDIAL INFARCTION**

(30) Priority: 18.01.2021 KR 20210006949
(71) Applicant: Regen Innopharm Inc., Seoul 06591 (KR)
(72) Inventor: OH, Il Hoan, Seoul 06084 (KR); PARK, Hun Jun, Seoul 06276 (KR); PARK, Bong Woo, Seoul 05678 (KR)
(74) Representative: Isarpatent
(86) International application number: PCT/KR2022/000900
(87) International publication number: WO 2022/154636

(57) **Abstract**

The present invention relates to a use of a miR-145 inhibitor in preventing aggravation of myocardial infarction or treating myocardial infarction. A miR-145 antagomir reduces ischemic myocardial injury and necrosis upon myocardial infarction and increases the regeneration of myocardial tissues, thereby exhibiting a regenerative therapeutic effect after onset of myocardial infarction. Even when administered alone, the miR-145 antagomir exhibits a similar level of therapeutic effect to that when administered in combination with a miR-124 antagomir, and particularly, brings about a remarkable therapeutic effect at the initial stage (acute phase) within several days after onset of myocardial infarction. Thus, the antagomir can be used for treating myocardial infarction.

## Description

### [Technical Field]

The present invention relates to a use of a mir145 inhibitor for the treatment of myocardial infarction.

### [Background Art]

Myocardial infarction is a fatal disease that causes myocardial necrosis and cardiac function deterioration due to blockage of coronary arteries supplying myocardial blood. Currently, there are about 16 million patients worldwide every year as of 2015, and one million myocardial infarction patients occur annually even in the United States alone (2016). Based on US statistics in 2011, the myocardial infarction was found to be one of the five most expensive diseases in medical costs during hospitalization, and it was found that hospitalization for 612,000 days and medical costs of 11 billion dollars were required (National inpatient Hospital Cost, 2011 report) (Statistical Brief #160 (ahrq.gov)). Various types of thrombolytic agents have been used to treat myocardial infarction, and percutaneous coronary intervention (PCI) has also been used, and coronary artery bypass surgery (CABG) is used to treat cases with underlying disease or invasion of multiple arteries (O'Connor et al., 2010). However, even after anticoagulants and coronary artery intervention techniques are applied, as the overall mortality rate is 10 to 12%, a new approach to treat myocardial infarction is required (Gershlick and More, 1998; Pollard, 2000; Williams and Morton, 1995).

Meanwhile, miRNA is one of non-coding regulatory RNAs, and serves to mainly degrade target mRNA or bind to mRNA to inhibit protein synthesis. Since miRNA was first discovered in *Caenorhabditis elegans* in 1993, various miRNAs have been discovered, and it has been found that these miRNAs played an important role in regulating cell function and fate through the regulation of cell gene expression (Elbashir et al., 2001; Lagos-Quintana et al., 2001).

Accordingly, the present inventors attempted a miRNA-based therapeutic approach for heart regeneration treatment of myocardial infarction, and as a result, found that the miRNA-based therapeutic approach may be used for myocardial regeneration and treatment of myocardial infarction.

### [Disclosure]

### [Technical Problem]

An object of the present invention is to provide a pharmaceutical composition for the treatment of myocardial infarction.

In addition, another object of the present invention is to provide a pharmaceutical composition for restoring a cardiac function.

In addition, yet another object of the present invention is to provide a pharmaceutical composition for palliating symptoms of dilative heart failure.

In addition, still another object of the present invention is to provide a pharmaceutical composition for reducing myocardial tissue lesions.

In addition, still yet another object of the present invention is to provide a method for treating myocardial infarction.

In addition, still yet another object of the present invention is to provide a use of a mir145 inhibitor to be used for preparing a pharmaceutical composition for the prevention and treatment of myocardial infarction.

### [Technical Solution]

One aspect of the present invention provides a pharmaceutical composition including a mir145 inhibitor as an active ingredient for the treatment of myocardial infarction.

In addition, another aspect of the present invention provides a pharmaceutical composition including a mir145 inhibitor as an active ingredient for restoring a cardiac function.

In addition, yet another aspect of the present invention provides a pharmaceutical composition including a mir145 inhibitor as an active ingredient for palliating symptoms of dilative heart failure.

In addition, still another aspect of the present invention provides a pharmaceutical composition including a mir145 inhibitor as an active ingredient for reducing myocardial tissue lesions.

In addition, still yet another aspect of the present invention provides a method for treating myocardial infarction including administering a mir145 inhibitor in a pharmaceutically effective amount to a subject suffering from myocardial infarction.

In addition, still yet another aspect of the present invention provides a use of a mir145 inhibitor to be used for preparing a pharmaceutical composition for the prevention and treatment of myocardial infarction.

### [Advantageous Effects]

According to the present invention, a miR-145 antagomir reduces ischemic myocardial injury and necrosis upon myocardial infarction and increases the regeneration of myocardial tissues, thereby exhibiting a regenerative therapeutic effect after onset of myocardial infarction. Even when administered alone, the miR-145 antagomir exhibits a similar level of therapeutic effect to that when administered in combination with a miR-124 antagomir, and particularly, brings about a remarkable therapeutic effect at the initial stage (acute phase) within several days after onset of myocardial infarction. Thus, the antagomir may be used for treating myocardial infarction.

### [Description of Drawings]

FIG. 1 is a diagram illustrating a result of screening a miRNA group capable of increasing an EMT gradient known to be involved in tissue regeneration among miRNAs differentially expressed in a 3D culture and a 2D culture and a miRNA group capable of regulating the expression of genes related to stemness.
FIG. 2 is a diagram illustrating a miRNA group targeting the EMT gradient and a miRNA group targeting pluripotency genes.
FIG. 3 is a diagram confirming induction of expression of EMT-related genes and pluripotency-related genes according to antagomir combinations (145+124, 145+203a, 145+124+34a, and 145+124+106b) for selected miRNA groups:
   145: miR-145-5p;
   124: miR-124-3p;
   203a: miR 203a-3p;
   34a: miR 34a-5P; and
   106b: miR 106b.
FIG. 4 is a diagram illustrating a process of preparing a myocardial infarction animal model and administering a drug.
FIG. 5 is a diagram confirming cardiac function improvement effects of an EMT-related antagomir to miR124-3p and a pluripotency-related antagomir to miR-145-5p by ultrasound.
FIG. 6 is a diagram confirming cardiac contractility by measuring left ventricular ejection fraction (% LVEF) and fractional shortening (% FS) and cardiac function improvement effects by measuring left ventricular internal systolic diameter (LVISd) and left ventricular internal diastolic diameter (LVIDd) with respect to an antagomir to miR124-3p and an antagomir to miR-145-5p:
   miR124: antagomir to miR124-3p; and
   miR145: antagomir to miR-145-5p.
FIG. 7 is a diagram confirming changes in motility of the myocardial wall by a combined administration of an antagomir to miR124-3p and an antagomir to miR-145-5p through echocardiography:
   miR12/14: Combined (concomitant) administration of antagomir to miR124-3p and antagomir to miR-145-5p.
FIG. 8 is a diagram confirming a cardiac function recovery effect by a combined administration of an antagomir to miR124-3p and an antagomir to miR-145-5p:
   124/145: Combined (concomitant) administration of antagomir to miR124-3p and antagomir to miR-145-5p.
FIG. 9 is a diagram confirming a myocardial tissue lesion reduction effect by histological analysis by a combined administration of an antagomir to miR124-3p and an antagomir to miR-145-5p:
   CHP: red;
   TNNT2: green;
   DAPI: blue color; and
   NC: negative control;
   miR 124/145 or 124/145: Combined (concomitant) administration of antagomir to miR124-3p and antagomir to miR-145-5p.
FIG. 10 is a diagram illustrating a cardiac function recovery effect in a myocardial infarction model according to a mixing ratio (1 : 1, 1/3 : 1 and 1/2 : 1) of an antagomir to miR124-3p and an antagomir to miR-145-5p administered in combination.
FIG. 11 is a diagram confirming changes in capillary density by histological analysis in a myocardial infarction model according to a mixing ratio (1 : 1, 1/3 : 1 and 1/2 : 1) of an antagomir to miR124-3p and an antagomir to miR-145-5p administered in combination.
FIG. 12 is a diagram confirming the fibrosis level and viable myocardium number according to a mixing ratio (1 : 1, 1/3 : 1 and 1/2 : 1) of an antagomir to miR124-3p and an antagomir to miR-145-5p administered in combination.
FIG. 13 is a diagram comparing a cardiac function recovery effect according to single/combined administration of an antagomir to miRNA 124 and an antagomir to miRNA 145:
   124+145: antagomir to miR124-3p and antagomir to miR-145-5p;
   miR124: antagomir to miR124-3p; and
   miR145: antagomir to miR-145-5p.
FIG. 14 is a diagram confirming changes in capillary density and viable myocardium number by histological analysis according to single/combined administration of an antagomir to miRNA 124 and an antagomir to miRNA 145:
   miR124: antagomir to miR124-3p;
   miR145: antagomir to miR-145-5p; and
   124/145: Combined (concomitant) administration of antagomir to miR124-3p and antagomir to miR-145-5p.
FIG. 15 is a diagram comparing myocardial fibrosis levels according to single/combined administration of an antagomir to miRNA 124 and an antagomir to miRNA 145:
   scramble: negative control;
   miR124: antagomir to miR124-3p;
   miR145: antagomir to miR-145-5p;
   124/145: Combined (concomitant) administration of antagomir to miR124-3p and antagomir to miR-145-5p; and
   12/14: Combined (concomitant) administration of antagomir to miR124-3p and antagomir to miR-145-5p.

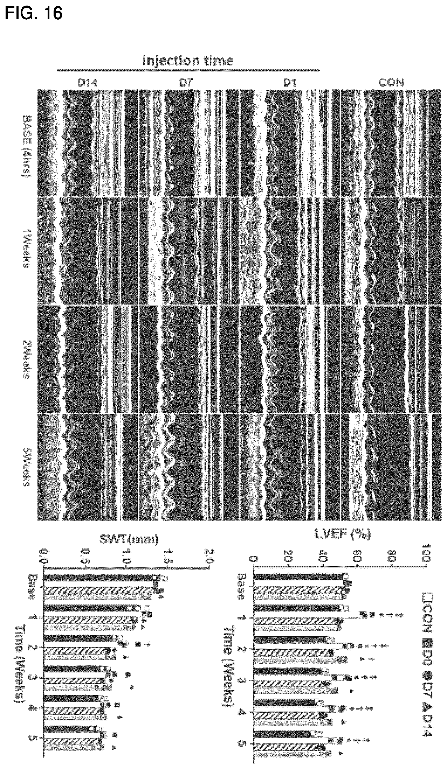

FIG. 16 is a diagram confirming a therapeutic effect of myocardial infarction according to an administration time of an antagomir to miRNA-145:
   D1: Administration of antagomir to miRNA-145 after 1 day of myocardial infarction;
   D7: Administration of antagomir to miRNA-145 after 7 days of myocardial infarction; and
   D14: Administration of antagomir to miRNA-145 after 14 days of myocardial infarction.

### [Modes of the Invention]

Hereinafter, exemplary embodiments of the present invention will be described in detail with reference to the accompanying drawings. However, the following exemplary embodiments are presented as examples for the present invention, and when it is determined that a detailed description of well-known technologies or configurations known to those skilled in the art may unnecessarily obscure the gist of the present invention, the detailed description thereof may be omitted, and the present invention is not limited thereto. Various modifications and applications of the present invention are possible within the description of claims to be described below and the equivalent scope interpreted therefrom.

Terminologies used herein are terminologies used to properly express examples of the present invention, which may vary according to a user, an operator's intention, or customs in the art to which the present invention pertains. Therefore, these terminologies used herein will be defined based on the contents throughout the specification. Throughout the specification, unless explicitly described to the contrary, when a certain part "comprises" a certain component, it will be understood to imply the inclusion of stated elements but not the exclusion of any other elements.

All technical terms used in the present invention, unless otherwise defined, have the meaning as commonly understood by those skilled in the related art of the present invention. In addition, although preferred methods and samples are described herein, similar or equivalent methods and samples thereto are also included in the scope of the present invention. The contents of all publications disclosed as references in this specification are incorporated in the present invention.

In one aspect, the present invention relates to a pharmaceutical composition including a mir145 inhibitor as an active ingredient for the treatment of myocardial infarction.

In an exemplary embodiment, mir145 may be mir-145-5p, and mir-145-5p may include a nucleotide sequence represented by SEQ ID NO: 1.

In an exemplary embodiment, the mir145 inhibitor may be at least one selected from the group consisting of an inhibitory RNA molecule, an antagonist microRNA, an enzymatic RNA molecule, and an anti-miRNA antibody, and may be a nucleic acid molecule that specifically binds to mir145. In addition, the mir145 inhibitor may be at least one selected from the group consisting of an antisense oligonucleotide, an antagomir, a ribozyme, an aptamer, siRNA, miRNA, short hairpin RNA (shRNA), peptide nucleic acid (PNA) and locked nucleic acid (LNA), more preferably an antagomir, and most preferably anti-mir145 represented by SEQ ID NO: 4.

In an exemplary embodiment, the term "nucleic acid" includes a polynucleotide, an oligonucleotide, DNA, RNA, and analogs thereof, and derivatives thereof, and for example, a peptide nucleic acid (PNA) or mixtures thereof. In addition, the nucleic acid may be a single or double strand.

The term "miR" used herein refers to "micro RNA" and may be used interchangeably.

As used herein, the term "antagomir" is a chemically modified single-stranded oligonucleotide, used for silencing endogenous miRNA, and has a sequence complementary to a target sequence. In the present invention, the antagomir was used by modifying a complementary RNA sequence of miRNA into a hydroxyprolinol-linked cholesterol solid support and 2'-OMe phosphoramidites so as to effectively bind to miRNA and inhibit miRNA (see Kru tzfeldt, J., Rajewsky, N., Braich, R., Rajeev, K.G., Tuschl, T., Manoharan, M., and Stoffel, M. (2005). Silencing of microRNAs in vivo with "antagomirs." Nature 438, 685-689), but is not limited thereto.

As used herein, the term "complementary" means that an antisense oligonucleotide is sufficiently complementary to selectively hybridize to a miRNA target under predetermined hybridization or annealing conditions, preferably physiological conditions, and has a meaning encompassing both partly or partially substantially complementary and completely complementary. The substantially complementary, but not completely complementary, refers to complementarity enough to exhibit an effect according to the present invention, that is, an effect sufficient to interfere with the expression of the miRNA by binding to a target sequence.

In an exemplary embodiment, the inhibitor may include a nucleic acid encoding and expressing a nucleic acid molecule that specifically binds to the miRNA, may be a plasmid including the nucleic acid, and may be administered in the form of a plasmid, a vector, antisense, shRNA, oligo, etc. used in the general technical art.

In an exemplary embodiment, the composition of the present invention may further include a mir124 inhibitor, and the mir124 inhibitor may be an antagomir to mir124 and may include a nucleotide sequence represented by SEQ ID NO: 5 or 6.

In an exemplary embodiment, the composition of the present invention may further include one or more inhibitors selected from the group consisting of a miR 203a inhibitor, a miR181 inhibitor, a miR128 inhibitor, a miR-128 inhibitor, a mir106b inhibitor, and a miR34a inhibitor.

In an exemplary embodiment, the composition of the present invention may be administered in the presence of ischemic myocardial necrosis or acute inflammation.

In an exemplary embodiment, the myocardial infarction may be ischemic myocardial necrosis or acute phase (or initial stage) myocardial infarction with acute inflammation, and the acute phase may be within 1 week of onset of myocardial infarction.

In an exemplary embodiment, the miR 124 inhibitor or miR 145 inhibitor may be small RNA.

The small RNA (hereinafter referred to as "sRNA") refers to ribonucleic acid with a length of about 17 to 25 nucleotides (nucleotide: hereinafter referred to as "nt") that serves to regulate gene expression in vivo. The sRNA is largely classified into microRNA (miR: hereinafter referred to as "miRNA") and small interfering RNA (hereinafter referred to as "siRNA") according to its production method. The miRNA is generated from hairpin RNA that partially forms a double helix, and the siRNA is derived from long double-strand RNA (hereinafter referred to as "dsRNA"). In general, the sRNA that plays an important role in various regulatory processes in vivo is miRNA, and the miRNA is a single-stranded RNA (hereinafter referred to as "ssRNA") molecule with a length of 19 to 25 nt and produced by an endogenous hairpin-shaped transcript (Bartel, D.P., Cell 116:281-297, 2004; Kim, V.N., Mol. Cells. 19:1-15, 2005). The miRNAs act as a post-transcriptional gene suppressor by complementarily binding to 3' untranslated regions (UTRs) of target mRNA, and inhibits target genes by inducing translational repression and mRNA destabilization. The miRNA plays an important role in various processes such as development, differentiation, proliferation, apoptosis and metabolism.

In addition, miRNA biosynthesis is initiated through transcription by RNA polymerase II (Cai. X., et al., RNA 10:1957-1966, 2004; Kim, V.N. Nat. Rev. Mol. Cell. Biol. 6:376-385, 2005; Lee, Y., et al., EMBO J 21:4663-4670,2002; Lee, Y., et al., EMBO J 23:4051-4060, 2004). Primary miRNA (primary microRNA: hereinafter referred to as "pri-miRNA") usually exceed several Kb in length and includes a 5' cap and a poly A tail. The pri-miRNA is first cleaved by a complex called a microprocessor consisting of ribocuclease III, Drosha (Lee, Y., et al., Nature 425:415-419, 2003) and its sub-element DGCR8 (Gregory, R.I. et al., Nature 432:235-240, 2004; Han, J., et al., Genes Dev. 18:3016-3027, 2004; Landthaler, M., et al., Curr. Biol. 14:2162-2167, 2004) to be isolated into a hairpin-structured precursor (pre-miRNA) of about 65 nt. The pre-miRNA process is a central step in miRNA biogenesis and is defined as a process of producing one end of a molecule containing an mRNA sequence in long pre-miRNA. The pre-miRNA produced after the initiation process is transported to the cytoplasm by a nuclear transport factor exportin-5 (Exp5) (Bohnsack, M.T., et al., RNA 10:185-191, 2004; Lund, E., et al., Science 303:95-98, 2004; Yi, R., et al., Genes Dev. 17:3011-3016, 2003). In the cytoplasm, Dicer, an intracytoplasmic RNase III type protein, cleaves the transferred pre-miRNA to generate miRNA double strands of about 22 nt. One strand of the Dicer product is present in the cytoplasm as mature miRNA and assembled with an effector complex called microribonucleoprotein (miRNP) or miRNA-induced silencing complex (miRISC) (Khvorova, A., et al., Cell 115:209-216, 2003; Schwarz, D.S., et al., Cell 115:199-208, 2003). RNAi, a gene silencing mechanism by sRNA, has been used as an effective gene research tool in a mammalian system. Effective and stable gene knockdown occurs while expressed as small hairpin RNA (hereinafter referred to as "shRNA") and processed into small interfering RNA (siRNA). A recent breakthrough in RNAi technology have been achieved by constructing shRNA expression cassettes that mimic natural miRNA genes (Dickins, R.A., et al., Nat. Genet. 37:1289-1295, 2005; Silva, J.M. et al., Nat. Genet. 37:1281-1288, 2005: Zeng, Y., et al., Mol. Cell 9:1327-1333. 2002). shRNA based on miRNA regulated by an RNA polymerase II promoter effectively and stably induces gene silencing regulation in cultured cells such as an animal model.

The term "treatment" as used herein, unless otherwise stated, means reversing and palliating a disease or disorder to which the treatment is applied, or one or more symptoms of the disease or disorder, preventing its progression, or preventing aggravation after onset. The term "treatment" used herein refers to an act of treating myocardial infarction. Accordingly, treatment or therapy for myocardial infarction in a mammal may include one or more of the following:
(1) inhibiting the development of myocardial infarction, i.e., arrest its development;
(2) preventing aggravation after onset of myocardial infarction;
(3) reducing myocardial infarction;
(4) preventing recurrence of myocardial infarction; and
(5) palliating symptoms of myocardial infarction.

As used herein, the term "mammal" refers to a mammal that is a subject to be treated, observed or experimented, and preferably refers to a human.

If a recipient animal is able to tolerate administration of the composition, or administration of the composition to the animal is suitable, it is indicated that the composition is "pharmaceutically or physiologically acceptable". The agent has been administered in a "therapeutically effective amount" when the administered amount is physiologically significant. If the presence of the agent results in a physiologically detectable change in the recipient patient, the agent is physiologically significant.

The therapeutically effective dose of the composition of the present invention may vary depending on many factors, for example, an administration method, a target site, a condition of a patient, and the like. Accordingly, when used in the human body, the dose should be determined as an appropriate amount in consideration of both safety and efficiency. It is also possible to estimate the amount used in humans from the effective dose determined through animal experiments. These matters to be considered when determining the effective dose are described in, for example, Hardman and Limbird, eds., Goodman and Gilman's The Pharmacological Basis of Therapeutics, 10th ed. (2001), Pergamon Press; and E.W. Martin ed., Remington's Pharmaceutical Sciences, 18th ed. (1990), Mack Publishing Co.

The pharmaceutical composition of the present invention is administered in a pharmaceutically effective dose. The term "pharmaceutically effective dose" used herein refers to an amount enough to treat the disease at a reasonable benefit/risk ratio applicable to medical treatment and not causing side effects. An effective dose level may be determined according to factors including the health condition of a patient, the type and severity of a disease, the activity of a drug, the sensitivity to a drug, a method of administration, a time of administration, a route of administration, an emission rate, duration of treatment, and factors including drugs used in combination or simultaneously, and other factors well-known in the medical field. The composition of the present invention may be administered as an individual therapeutic agent or in combination with other therapeutic agents, and may be administered sequentially or simultaneously with existing therapeutic agents, and may be administered singly or multiply. It is important to administer an amount capable of obtaining a maximum effect with a minimal amount without side effects by considering all the factors, which may be easily determined by those skilled in the art.

The composition of the present invention may include a carrier, a diluent, an excipient, or a combination of two or more thereof, which are commonly used in biological agents. The pharmaceutically acceptable carrier is not particularly limited as long as the carrier is suitable for in vivo delivery of the composition, and may be used by combining, for example, compounds described in Merck Index, 13th ed., Merck & Co. Inc., saline, sterile water, a Ringer's solution, buffered saline, a dextrose solution, a maltodextrin solution, glycerol, ethanol, and one or more of these components, and if necessary, other conventional additives such as an antioxidant, a buffer, and a bacteriostat may be added. In addition, the pharmaceutical composition of the present invention may be prepared in injectable formulations such as an aqueous solution, a suspension, and an emulsion, pills, capsules, granules, or tablets by further adding a diluent, a dispersant, a surfactant, a binder, and a lubricant. Furthermore, the composition may be prepared preferably according to each disease or ingredient using as a suitable method in the art or a method disclosed in Remington's Pharmaceutical Science (Mack Publishing Company, Easton PA, 18th, 1990).

The pharmaceutical composition according to the present invention may be formulated and used in the form of oral formulations such as powders, granules, tablets, capsules, suspensions, emulsions, syrups, aerosols, etc., external preparations, suppositories, and sterile injectable solutions according to a conventional method.

As used herein, the term "pharmaceutically acceptable" refers to exhibiting non-toxic properties to cells or humans exposed to the composition.

The pharmaceutical composition of the present invention may further include pharmaceutically acceptable additives. At this time, the pharmaceutically acceptable additive may use starch, gelatinized starch, microcrystalline cellulose, lactose, povidone, colloidal silicon dioxide, calcium hydrogen phosphate, lactose, mannitol, syrup, gum arabic, pregelatinized starch, corn starch, powdered cellulose, hydroxypropyl cellulose, Opadry, sodium starch glycolate, lead carnauba, synthetic aluminum silicate, stearic acid, magnesium stearate, aluminum stearate, calcium stearate, sucrose, dextrose, sorbitol, talc, and the like. The pharmaceutically acceptable additive according to the present invention is preferably included in an amount of 0.1 part by weight to 90 parts by weight based on the composition, but is not limited thereto.

As used herein, the term "administration" means providing a predetermined substance to a patient by any suitable method, and the pharmaceutical composition may be administered parenterally (e.g., intravenously, subcutaneously, intraperitoneally or topically applied) or orally according to a desired method, and the dose range may vary depending on the body weight, age, sex, and health condition of a patient, a diet, an administration time, an administration method, an excretion rate, and the severity of a disease.

The composition of the present invention may be administered parenterally (for example, intravenously, subcutaneously, intraperitoneally or topically applied) or orally, depending on a desired method, and the dosage is selected in consideration of the subject's age, weight, sex, physical condition, etc. It is obvious that the concentration of the active ingredient included in the pharmaceutical composition may be variously selected according to a subject, and the active ingredient is preferably included in the pharmaceutical composition at a concentration of 0.01 µg/ml to 50 mg/ml. When the concentration is less than 0.01 µg/ml, pharmacological activity may not be shown, and when the concentration exceeds 50 mg/ml, toxicity to the human body may be exhibited.

The pharmaceutical composition of the present invention may be formulated into various oral or parenteral dosage forms. Formulations for oral administration include, for example, tablets, pills, hard and soft capsules, solutions, suspensions, emulsifiers, syrups, granules, etc., and these formulations may further include diluents (e.g., lactose, dextrose, sucrose, mannitol, sorbitol, cellulose and/or glycine), lubricants (e.g., silica, talc, stearic acid and its magnesium or calcium salts and/or polyethylene glycol), in addition to the active ingredient. Further, the tablets may contain a binder such as magnesium aluminum silicate, starch paste, gelatin, tragacanth, methylcellulose, sodium carboxymethylcellulose and/or polyvinylpyrrolidine, and in some cases, may contain disintegrants or effervescent mixtures such as starch, agar, alginic acid or its sodium salt and/or an absorbent, a coloring agent, a flavoring agent and a sweetening agent. The formulations may be prepared by conventional mixing, granulating or coating methods. In addition, a typical formulation for parenteral administration is an injection formulation, and as a solvent for the injection formulation, water, a Ringer's solution, isotonic physiological saline or a suspension may be used. Sterile fixed oils of the injection formulation may be used as a solvent or suspension medium, and any non-irritating fixed oil including mono- and di-glycerides may be used for this purpose. In addition, the injection formulation may use fatty acids such as oleic acid.

In the present invention, the term "prevention" refers to all actions that inhibit or delay the onset, aggravation, and recurrence of myocardial infarction by administration of the pharmaceutical composition according to the present invention.

In one aspect, the present invention relates to a pharmaceutical composition including a mir145 inhibitor as an active ingredient for restoring a cardiac function in myocardial infarction.

In an exemplary embodiment, the pharmaceutical composition may increase the motility of the myocardial wall, cardiac output, left ventricular ejection fraction (% LVEF) and fractional shortening (% FS), and decrease left ventricular internal diastolic diameter (LVIDd) and left ventricular internal systolic diameter (LVISd).

In one aspect, the present invention relates to a pharmaceutical composition including a mir145 inhibitor as an active ingredient for palliating dilative heart failure symptoms by myocardial remodeling and dilatation in myocardial infarction.

In an exemplary embodiment, the composition may be used as a pharmaceutical composition for the treatment of dilative heart failure.

In one aspect, the present invention relates to a pharmaceutical composition including a mir145 inhibitor as an active ingredient for reducing myocardial tissue lesions in myocardial infarction.

In an exemplary embodiment, the composition may prevent myocardial necrosis and injury, promote regeneration of normal myocardium, increase capillary density and viable myocardium number, and suppress myocardial fibrosis.

In one aspect, the present invention provides a method for treating myocardial infarction including administering a mir145 inhibitor in a pharmaceutically effective amount to a subject suffering from myocardial infarction.

In an exemplary embodiment, the myocardial infarction may be acute phase myocardial infarction.

In one aspect, the present invention provides a use of a mir145 inhibitor to be used for preparing a pharmaceutical composition for the prevention and treatment of myocardial infarction.

In an exemplary embodiment, the myocardial infarction may be acute phase myocardial infarction.

Hereinafter, the present invention will be described in more detail with reference to the following Examples. However, the following Examples are only intended to embody the contents of the present invention, and the present invention is not limited thereto.

### Example 1. Screening and identification of EMT gradient and pluripotency-related miRNAs

### 1-1. Screening of EMT gradient increasing miRNA and stem cell gene expression regulating miRNA

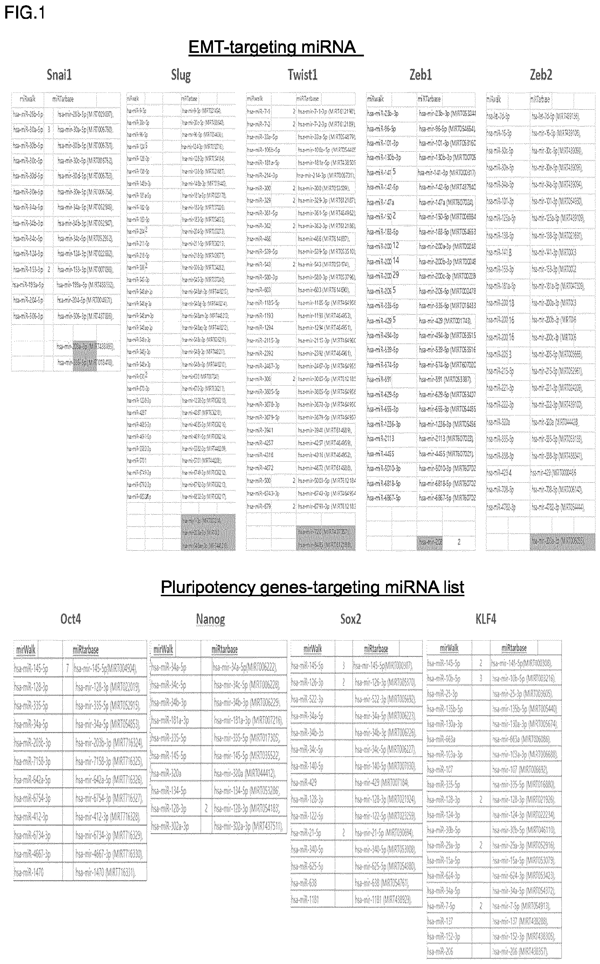

In a previous study by the present inventors, it was confirmed that the regenerative capacity of cells was increased through 3D culture, and among them, changes in miRNAs related to epithelial-mesenchymal transition (EMT) were characteristically observed to increase the EMT gradient. Accordingly, among miRNAs differentially expressed in 3D culture and 2D culture, miRNA groups capable of increasing the EMT gradient were screened, and target genes of the differentially expressed miRNAs were tracked to a miRNA database (miRWalk2.0 and miRtargbase). In addition, miRNAs regulating the expression of genes related to sternness in cells were screened through a similar miR database (FIG. 1).

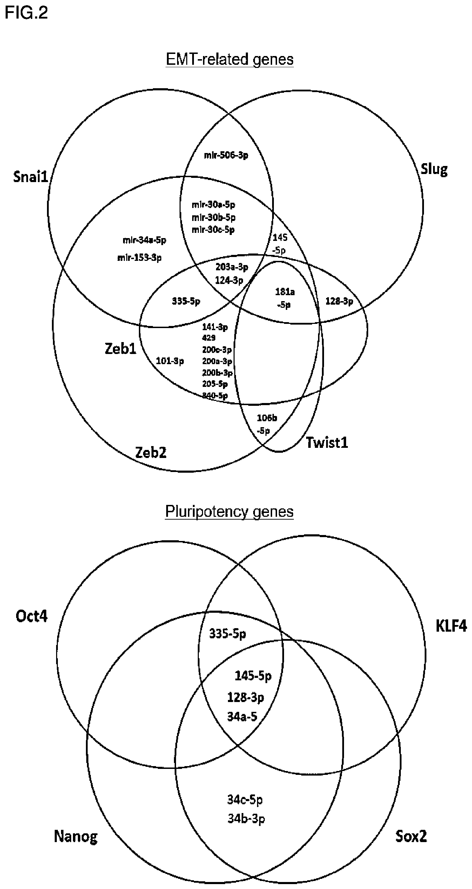

As a result, it was confirmed that miR 203a-3p, miR124-3p, miR181-5p and miR128-3p could promote the EMT gradient, and it was confirmed that miRNAs such as miR145-5p, miR-128-3p, and miR 34a-5P commonly targeted pluripotency genes oct-4, nanog, Klf-4, and Sox-2 (FIG. 2).

### 1-2. Confirmation of increased expression of EMT gradient and pluripotency genes of antagomirs

In order to confirm whether an antagomir combination of miRNA candidates selected in Example 1-1 may increase the expression of genes related to EMT gradient and pluripotency required for cell regeneration, gene expression patterns for each combination were compared. Specifically, antagomirs (Table 1) of each miRNA selected above were ordered from Shanghai Gene Pharma Co., Ltd., and MSCs derived from human bone marrow were transfected with various combinations of miR-antagomirs, respectively, and then the expression levels of EMT gradient-related genes Snail, Slug, Twist1, Zeb1, and Zeb2, and pluripotency-related genes Oct4, Nanog, Sox2, and KLF4 were confirmed by qRT-PCR.

**[Table 1]**

| | Type | Sequence (5'->3') | SEQ ID NO: |
|---|---|---|---|
| Human miR-145-5p | Mature hsa-miR-145-5p | GUCCAGUUUUCCCAGG AAUCCCU | 1 |
| | hsa-miR-145-5p antagomir | AGGGAUUCCUGGGAAA ACUGGAC | 4 |
| Human miR-124-3p | Mature hsa-miR-124-3p | UAAGGCACGCGGUGAA UGCCAA | 2 |
| | hsa-miR-124-3p antagomir | UUGGCAUUCACCGCGU GCCUUA | 5 |
| Rat miR-124-3p | Mature rno-miR-124-3p | UAAGGCACGCGGUGAA UGCC | 3 |
| | mo-miR-124-3p antagomir | GGCAUUCACCGCGUGC CUUA | 6 |

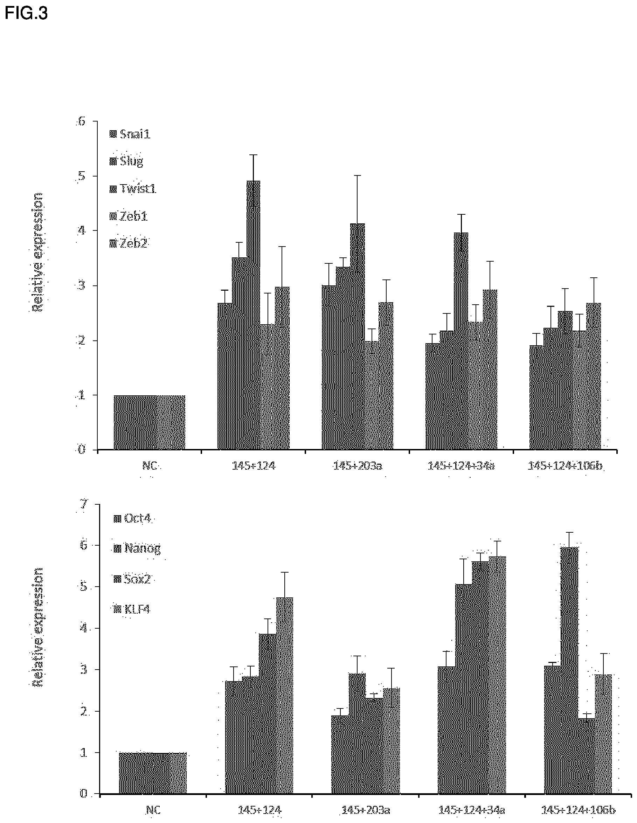

As a result, it was confirmed that among various combination candidates, 4 combinations of miR-antigomirs (145+124, 145+203a, 145+124+34a, and 145+124+106b) may significantly induce the expression of EMT-related genes and pluripotency genes compared to a control group (scrambled oligonucleotide: NC) (FIG. 3). Therefore, in order to confirm whether these miRNAs exhibited regeneration in the heart with myocardial infarction, a study was conducted to determine whether miR124-3p, one of the EMT-related miRNAs, and miR-145-5p, one of the miRNAs related to pluripotency, may show myocardial regeneration therapeutic effects independently or in combination.

### Example 2. Analysis of myocardial infarction therapeutic effect of antagomir

### 2-1. Construct of myocardial infarction animal model

Rats (Fischer 344, F344) were bred in an animal room of Catholic Univ. of Korea Songeui Campus and used for animal experiments after a stabilization period, and this study was conducted with the approval of the Animal Experiment Deliberative Committee of the Catholic Univ. of Korea Songeui Campus (Approval No. 2019-0058-01). The chest of the rat was cut and a left anterior descending artery (LAD) of the exposed heart coronary arteries was ligated, and then the ligation was removed to induce myocardial infarction by a myocardial infarction and ischemic reperfusion model to construct a myocardial infarction animal model. During the process of constructing the myocardial infarction animal model, upon recirculation by ligation removal of LAD, each miRNA antagomir was first injected into the myocardium around the infarct area with a syringe, and second injected into the myocardium after thoracotomy in the same manner, after 1 week of the induction of myocardial infarction.

### 2-2. Confirmation of cardiac function improvement effect

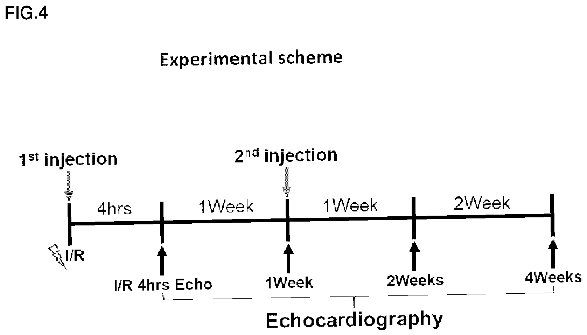

In order to confirm a therapeutic effect of myocardial infarction by antagomirs, changes in cardiac function were measured through echocardiography for a period from 1 to 4 weeks based on a time point of 4 hours after the induction of myocardial infarction (FIG. 4). In addition, cardiac contractility was evaluated through left ventricular ejection fraction (LVEF) and left ventricular fractional shortening. In addition, changes in heart size due to dilative heart failure were quantitatively evaluated through left ventricular internal systolic diameter (LVISd) and left ventricular internal diastolic diameter (LVIDd).

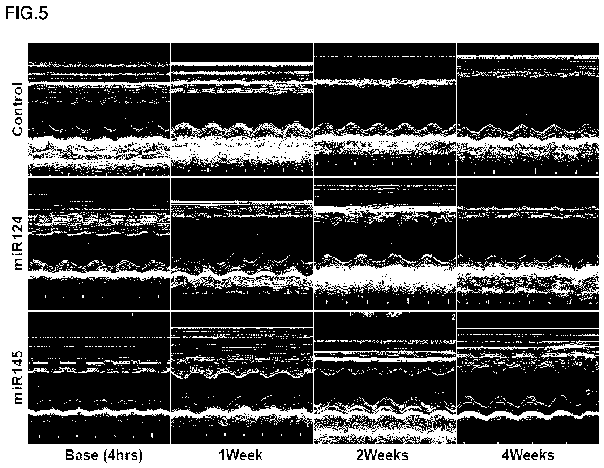

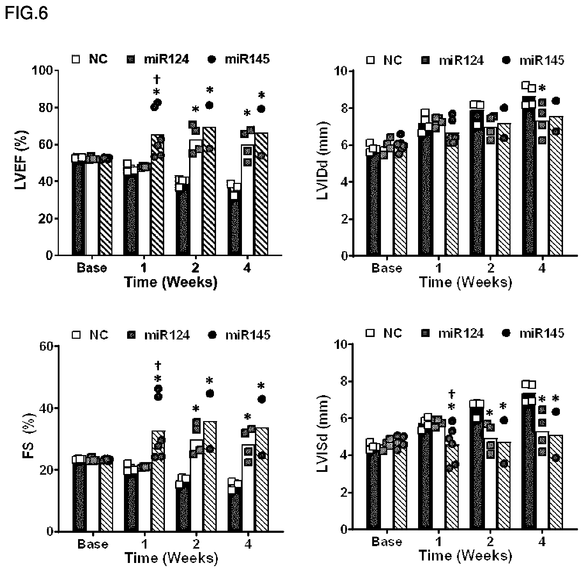

As a result, when an antagomir to miR-145, a miR-145-5p inhibitor, was injected into the myocardium, significant increases in left ventricular ejection fraction (% LVEF) and fractional shortening (% FS) were observed from one week after injection, and it was shown that improvement in cardiac function was maintained up to post-infarct 4 weeks (FIGS. 5 and 6). On the other hand, when an antagomir to miR-124, a miR-124-3p inhibitor, was injected into the myocardium, no effect was shown after 1 week of injection, but from post-infarct 2 weeks, left ventricular ejection fraction and fractional shortening were increased significantly, and it was shown that the effect was maintained up to 4 weeks after infarction (FIGS. 5 and 6). In addition, it was confirmed that the internal diastolic diameter (LVIDd) and the internal systolic diameter (LVISd) were decreased significantly in a treated group compared to a control group together with an increase in cardiac output (FIGS. 5 and 6), so that the symptoms of dilative heart failure were palliated by myocardial remodeling and dilatation.

Through this, it was confirmed that an antagomir to miR-124 and an antagomir to miR-145 could independently induce recovery of cardiac function injured by myocardial infarction at different time points.

### Example 3. Analysis of myocardial infarction therapeutic effect of antagomir combination

### 3-1. Confirmation of cardiac function recovery effect

During the process of constructing the myocardial infarction animal model of Example 2-1, upon recycling by ligation removal of the LAD, each miRNA antagomir was directly injected into the myocardium around the infarct area by combining an antagomir to miR-124 and an antagomir to miR-145 with a syringe (combined administration), and then a cardiac function improvement effect was confirmed as in Example 2-2.

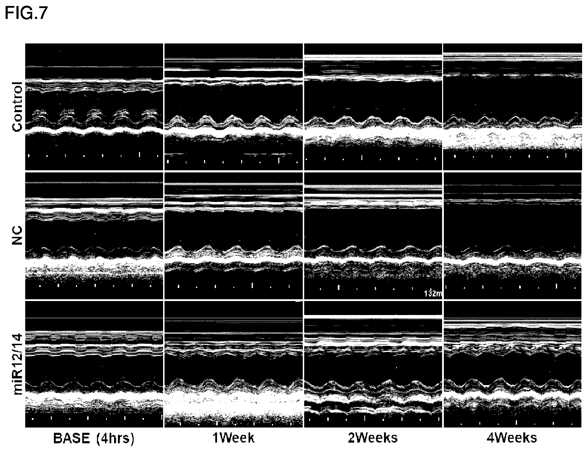

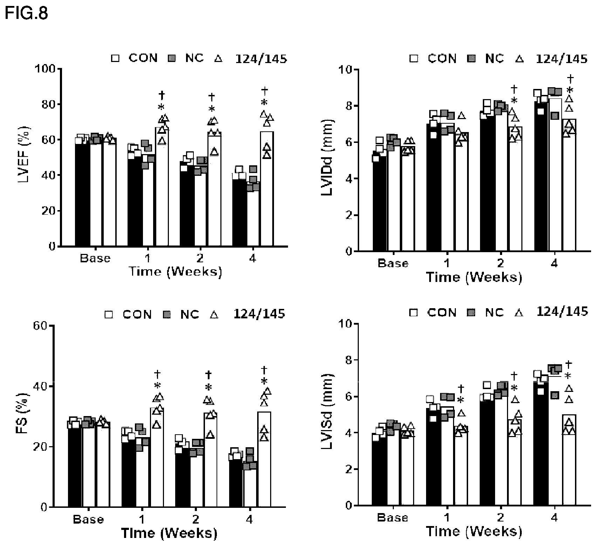

As a result, upon echocardiography, it was shown that the motility of the myocardial wall increased, and % LVEF and % FS were increased (FIG. 7 and 8). In addition, it was confirmed that the internal diastolic diameter (LVIDd) and the internal systolic diameter (LVISd) were decreased significantly in a treated group compared to a control group together with an increase in cardiac output, so that the symptoms of dilative heart failure were palliated by myocardial remodeling and dilatation (FIGS. 7 and 8).

Through this, it was confirmed that combined administration of the antagomir to miR-124 and the antagomir to miR-145 had an effect on restoring the cardiac function injured by myocardial infarction.

### 3-2. Myocardial tissue lesion reduction effect

To analyze the effect of treatment by the combined administration of the antagomir to miR-124 and the antagomir to miR-145 on myocardial tissue, by performing immunofluorescence staining, the degree of hybridization with collagen hybridizing peptide (CHP: red color) capable of measuring the degree of denaturation of collagen in cardiac muscle, troponin (TNNT2: green color) as normal myocardial tissue and nucleic acid (DAPI: blue color) were histologically analyzed. For quantitative result, the injury and remodeling of myocardial tissue were analyzed through Image J.

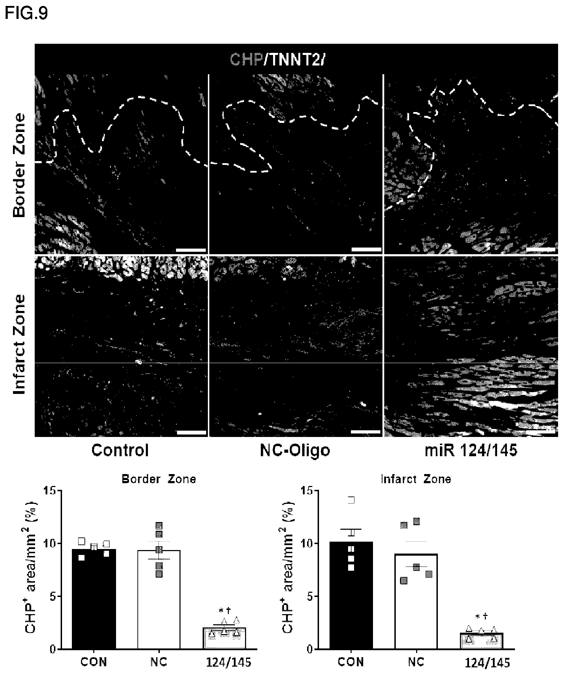

As a result, in a group co-administered with the antagomir to miR-124 and the antagomir to miR-145, as compared to a control group, it was confirmed that the myocardial lesion area (CHP-positive area; red) was significantly decreased, and the normal myocardial tissue (CHP-negative/TNN2-positive: green) was significantly increased (FIG. 9).

Accordingly, it may be seen that the combined administration of the antagomir to miR-124 and the antagomir to miR-145 prevents myocardial necrosis and injury, and promotes the regeneration of normal myocardium to promote the recovery of cardiac function after myocardial infarction.

### Example 4. Analysis of myocardial infarction therapeutic effect according to antagomir combination condition

### 4-1. Confirmation of effect according to antagomir mixing ratio

The myocardial infarction therapeutic effect was compared according to mixing ratios (1 : 1, 1/3 : 1 and 1/2 : 1) of the antagomir to miR-124 and the antagomir to miR-145 (ratio w/w of total administered dose (µg) of each antagomir).

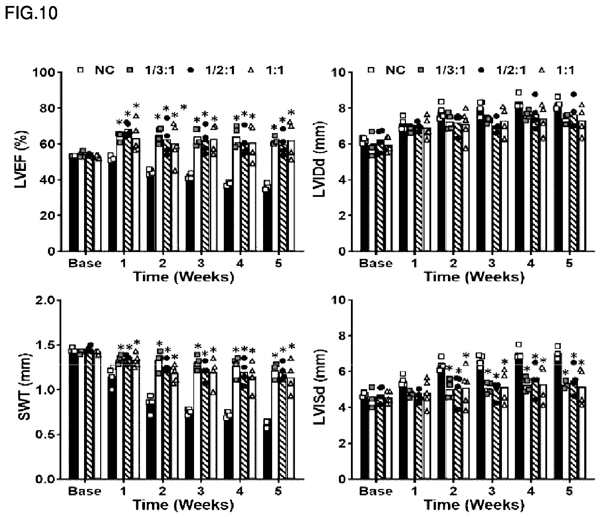

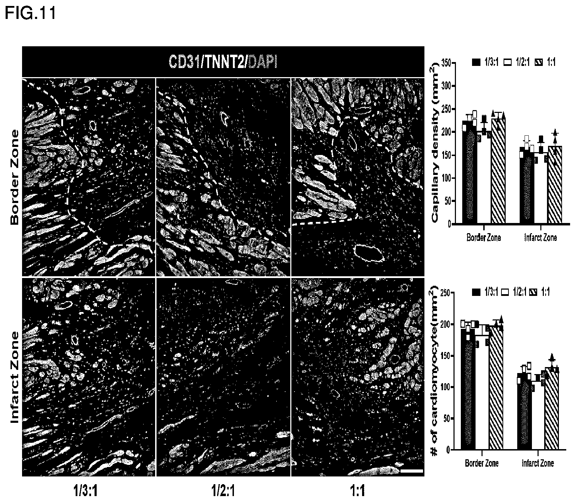

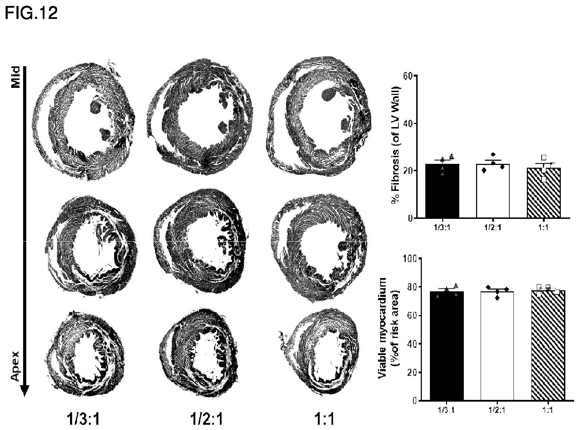

As a result, there was no significant difference in an increase in LVEF (%) by the combined administration of the two antagomirs in both the 1 : 1 mixed group and the 1/3 : 1 and 1/2 : 1 mixed groups, and even in other cardiac function evaluations (LVID and SWT), there was no significant difference between the three groups (FIG. 10). As a result of histological analysis, it was found that there was no difference in capillary density among the three groups (1/3 : 1, 1/2 : 1 and 1 : 1) (FIG. 11). Further, even in the result of confirming the fibrosis level and the viable myocardium number according to each mixing ratio, it was confirmed that there was no difference according to the three mixing ratios (FIG. 12).

### 4-2. Comparison of therapeutic effects of single and combined administration of antagomirs

Since the ratio of the antagomir to miRNA 124 in Example 4-3 did not significantly affect the overall treatment effect, the therapeutic effects of single administration of the antagomir to miRNA 124 and the antagomir to miRNA 145 were compared and analyzed with the combined administration of these combinations.

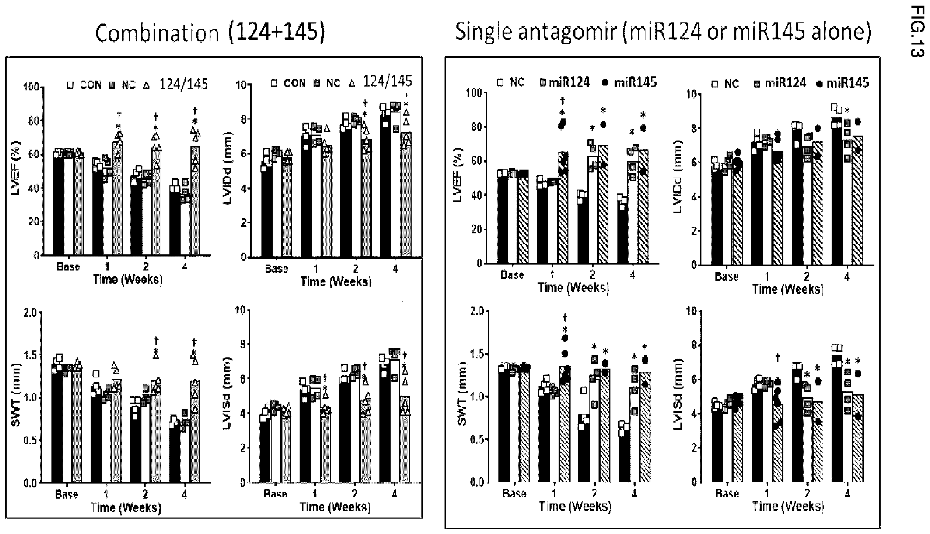

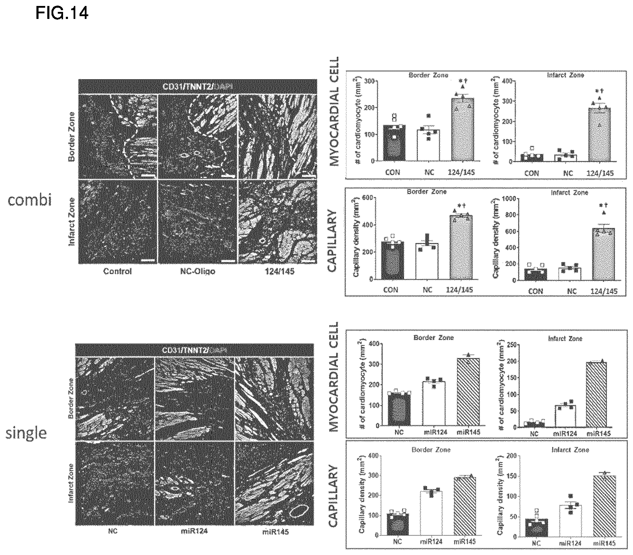

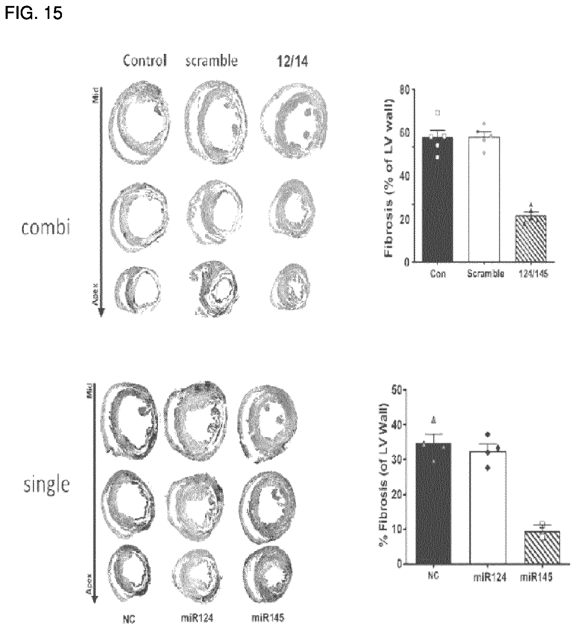

As a result of confirming the effect on the recovery of cardiac function, at 1 week after treatment for myocardial infarction, a miRNA 145-alone inhibitory group (group administered with antagomir to miR-145) exhibited a more pronounced cardiac function improvement (LVEF) effect than a miRNA-124-alone inhibitory group (group administered with antagomir to miR-124), and at 2 to 4 weeks, even in the miRNA-124-alone inhibitory group, the improved cardiac function improvement effect was exhibited compared to the control group (right side of FIG. 13). In addition, it was shown that the inhibitory effect of miR-124 or miR-145 alone was similar to that of the combination thereof (left vs right comparison in FIG. 13). Similarly, even in other cardiac function indices (SWT and LVID), the cardiac function improvement effect in each single administration group was observed according as the combined administration group (FIG. 13). On the other hand, as a result of histological analysis, the inhibition of miRNA-145 alone showed higher capillary density and viable myocardium number compared to inhibition of miRNA-124 alone, and it was confirmed that the effect was similar to that of the combination (124/145) thereof (FIG. 14). In addition, when analyzing myocardial fibrosis, the miR-145-alone inhibitory group showed an effect of significantly inhibiting myocardial fibrosis compared to the miR-124-alone inhibitory group, and it was confirmed that the effect was similar to the effect of these combinations (124/145) (FIG. 15).

Accordingly, it was confirmed that the myocardial infarction therapeutic effect by the combination (combined administration) of the antagomir to miRNA 124 and the antagomir to miRNA 145 may be reproduced by single administration of the antagomir to miRNA 145.

### Example 5. Analysis of therapeutic effect by administration time of antagomir to miRNA-145

In order to confirm the therapeutic effect of myocardial infarction according to an administration time of the antagomir to miRNA-145, a single dose of the antagomir to miRNA-145 was administered at various time points (days 0, 7 and 14) for 2 weeks after the onset of myocardial infarction, and the degree of cardiac function recovery was analyzed.

As a result, when the antagomir to miRNA-145 was injected after day 1 of the onset of myocardial infarction, despite a single administration, significant improvement in cardiac function (LVEF%) was shown compared to the control group, and when injected on day 7 or day 14, there was no significant difference compared to the control group (FIG. 16). Therefore, during a period when ischemic necrosis and inflammation were activated due to onset of acute myocardial infarction, the antagomir to miRNA-145 exhibited a therapeutic effect, but it was confirmed that the therapeutic effect was not significantly shown in a stationary phase when acute inflammation and the like disappeared.

Accordingly, it may be seen that the antagomir to miRNA-145 has a therapeutic window-specific effect that is limited to a few days after the onset of myocardial infarction, that is, in the initial stage of myocardial infarction (period of acute inflammation) to exhibit the effect, and does not show the therapeutic effect of myocardial infarction in tissues where homeostasis has been restored.

Through Examples above, when the miR-124 and miR-145 antagomirs were administered alone or in combination, the ischemic myocardial injury and necrosis upon myocardial infarction were decreased and at the same time, the regeneration of myocardial tissues was increased, thereby exhibiting a regenerative therapeutic effect of symptoms of myocardial infarction. Even when administered alone, the miR-145 antagomir exhibits a similar level of therapeutic effect to that when administered in combination with a miR-124 antagomir, and it was confirmed that the acute phase-specific therapeutic effect of showing a therapeutic effect was exhibited only at the initial stage (acute phase) within several days after onset of myocardial infarction.

## Claims

1. A pharmaceutical composition for the treatment of myocardial infarction comprising a mir145 inhibitor as an active ingredient.

2. The pharmaceutical composition for the treatment of myocardial infarction of claim 1, wherein mir145 is mir-145-5p.

3. The pharmaceutical composition for the treatment of myocardial infarction of claim 1, wherein the mir145 inhibitor is at least one selected from the group consisting of an inhibitory RNA molecule, a microRNA antagonist, an enzymatic RNA molecule, and an anti-miRNA antibody.

4. The pharmaceutical composition for the treatment of myocardial infarction of claim 1, wherein the mir145 inhibitor is at least one selected from the group consisting of antisense oligonucleotide, antagomir, ribozyme, aptamer, siRNA, miRNA, short hairpin RNA (shRNA), peptide nucleic acid (PNA) and locked nucleic acid (LNA), that specifically bind to mir145.

5. The pharmaceutical composition for the treatment of myocardial infarction of claim 1, wherein the mir145 inhibitor is anti-mir145 represented by SEQ ID NO: 4.

6. The pharmaceutical composition for the treatment of myocardial infarction of claim 1, further comprising a mir124 inhibitor.

7. The pharmaceutical composition for the treatment of myocardial infarction of claim 6, further comprising: at least one inhibitor selected from the group consisting of a miR 203a inhibitor, a miR181 inhibitor, a miR128 inhibitor, a miR-128 inhibitor, a miR106b inhibitor, and a miR34a inhibitor.

8. The pharmaceutical composition for the treatment of myocardial infarction of claim 1, wherein the pharmaceutical composition is administered in the presence of ischemic myocardial necrosis or acute inflammation.

9. The pharmaceutical composition for the treatment of myocardial infarction of claim 1, wherein the myocardial infarction is acute phase myocardial infarction.

10. The pharmaceutical composition for the treatment of myocardial infarction of claim 9, wherein the acute phase myocardial infarction is myocardial infarction in the presence of ischemic myocardial necrosis or acute inflammation.

11. A pharmaceutical composition for restoring a cardiac function in myocardial infarction comprising a mir145 inhibitor as an active ingredient.

12. The pharmaceutical composition of claim 11, wherein the pharmaceutical composition increases the motility of the myocardial wall, cardiac output, left ventricular ejection fraction (% LVEF) and fractional shortening (% FS), and decreases left ventricular internal diastolic diameter (LVIDd) and left ventricular internal systolic diameter (LVISd).

13. A pharmaceutical composition comprising a mir145 inhibitor as an active ingredient for palliating dilative heart failure symptoms by myocardial remodeling and dilatation in myocardial infarction.

14. A pharmaceutical composition comprising a mir145 inhibitor as an active ingredient for reducing myocardial tissue lesions in myocardial infarction.

15. The pharmaceutical composition of claim 14, wherein the pharmaceutical composition prevents myocardial necrosis and injury and promotes normal myocardial regeneration.

16. The pharmaceutical composition of claim 14, wherein the pharmaceutical composition increases capillary density and viable myocardium number.

17. The pharmaceutical composition of claim 14, wherein the pharmaceutical composition inhibits myocardial fibrosis.

18. A method for treating myocardial infarction comprising administering a mir145 inhibitor in a pharmaceutically effective amount to a subject suffering from myocardial infarction.

19. The method for treating myocardial infarction of claim 18, wherein the myocardial infarction is acute phase myocardial infarction.

20. A use of a mir145 inhibitor to be used for preparing a pharmaceutical composition for the prevention and treatment of myocardial infarction.
